# EUROPEAN PATENT APPLICATION

(11) **EP 4 389 170 A1**
(43) Date of publication of application: **26.06.2024**
(21) Application number: 22306998.0
(22) Date of filing: 22.12.2022
(51) Int. Cl.: A61M 5/145, A61M 5/315, A61M 5/168

(54) **MINI SYRINGE DRIVER**

(71) Applicant: Eveon, 38330 Montbonnot-Saint-Martin (FR)
(72) Inventor: BOUILLET, Adrien, 38330 Montbonnot-Saint-Martin (FR); CHEDRU, Clémence, 38330 Montbonnot-Saint-Martin (FR)
(74) Representative: Icosa

(57) **Abstract**

The present invention relates to a syringe driver (10) configured to automatically empty a mini-syringe (100) extending along an injection axis (X) and comprising a plunger (101) configured to translate inside the mini-syringe, said driver comprising:
- a pushing element (12) configured to cooperate with the plunger (101),
- a control unit (14) configured to operate and control the pushing element (12) in order to homogeneously empty the mini-syringe,
o an activable securing and detection system (16) presenting: a securing system (16b) configured to receive and secure the mini-syringe,
o a detection system (16c) configured to detect the presence and the correct positioning of the mini-syringe, configured to emit positive feedback when the mini-syringe is correctly secured.

The control unit is configured to wait for positive feedback from the detection system for launching the operation of the pushing element.

## Description

### FIELD OF INVENTION

The present invention relates to an automatic injection device for small syringes.

### BACKGROUND OF INVENTION

Several innovative therapies require high precision during injection in order to control, among other parameters, both dose administration and flow-rate. This is, for example, the case of nowadays gene therapies due to the high potential of immunization to the viral vector. This also can be the case for exosomes, requiring gentle administration, but also on standard drug delivery requiring specific site administration at highly reduced dose in order to avoid systemic toxicity and improve local action. Micro-dosing can be defined as the precise injection of a small volume, below 1mL (preferably below 100µL), with a precision of less than 5% of the dose to be administered.

In order to be able to administrate a micro-dose of drug, a micro-pumping mechanism is technically useful.

Micro-pumping mechanisms are promising as they reduce the size and energy consumption of dosing concepts and enable new therapies. However, there are only few examples of micropump based devices to be found on the market due to several challenges to solve regarding drug delivery specific to micro-dosing.

The required flow range depends strongly on the drug application, and the drug concentration: dealing with internal pressure, or enabling administration to small animals (e.g. rodents).

Further, any person skilled in the art is confronted to the fact that:
- "Dosing Accuracy and Precision Requirements" are not uniform: an example is diabetes therapy, in which most insulin-pump suppliers consider a ±5% deviation to be acceptable. However, micro-dosing system require high level of precision even though the dose is small: percentage deviation should not increase,
- Dosing stability: Changing environmental conditions should not influence the dosing accuracy to allow safe use at all times,
- Dosing Flexibility: In many applications there is a need to adapt the flowrate to the patients' need, however, depending on the actuation method, the range of applicable flow rates can be limited.

Moreover, it is necessary to provide a technical solution which does not involve the development of custom primary drug containers due to the complex regularity barrier for drug and device development. Thus, the global challenge relies on finding a new solution to technical challenges above, but using standard of the industry.

The industry standards for injection remain disposable plastic syringes. Those can hold volumes from 0.5mL to over 20ml. Such syringes respond to industry standard such as NF-EN-ISO7886-1. However, those syringes are not precise enough when it comes to administer micro-doses. 1mL syringes primary graduation are 1/10 and secondary graduation are 1:100. Considering applications of industrial standards, those syringes present primary graduations with volume tolerance of 5% if the expelled volume is greater than the half of syringe nominal capacity, or 1.5% of nominal volume + 2% of expelled volume if the expelled volume is bellow half of nominal capacity. Thus, such syringes can easily have primary graduation tolerances of about 10 to 50µL depending on the product brand, making them unsuitable to deliver volume below 0.1mL, or requiring precision up to 10µL (e.g. 110, or 125µL) *(*Le Daré et al, 2021, international journal of pharmaceutics 608*).*

To meet micro-dosing requirements with such low volume syringe (1mL as example), it is necessary to have a system which enables to improve overall precision. Administering 60µL with such device at 5% precision requires to improve overall precision from 30 to 3µL.

The aim of such device should also to be able to control injection flow rate in a range which would not be possible to control manually, as example, 20pL.min 30µL.min, 45µL.min, 60 µL.min, with an increment of 1 to 5µL.min.

It is known from document EP3880274, a system for micro-dose injection. This patent covers a system for injecting includes a syringe body having proximal and distal ends, a syringe interior, and a syringe flange at the proximal end thereof. The system also includes an injectable fluid disposed in the syringe interior. The system further includes a stopper member disposed in the syringe interior. Moreover, the system includes a plunger member coupled to the stopper member. In addition, the system includes a finger flange removably coupled to the syringe flange, the finger flange including a proximally directed screw. The system also includes a rotatable member disposed on the proximally directed screw, the rotatable member defining a rotatable member opening through which the plunger member is disposed and having an elastic latch disposed adjacent the rotatable member opening. However, if this system provides a solution for micro-dose range precision, it does not enable the control the flow rate. It also necessitates a high number of gestures and steps to be done, by hand, by a user.

The aim of this invention is therefore to enable the safe and pecise automated emptying of a loaded mini-syringe beyond the human precision rate corresponding to one graduation on a standard syringe while limitating as much as possible the human interaction and handling of the syringe before injection.

### SUMMARY

This invention thus relates to a syringe driver configured to empty, in an automatic way, a loaded mini-syringe extending along an injection axis and presenting an external wall with a diameter d, the mini-syringe further comprising a plunger configured to translate inside the external wall and also extending along said injection axis, said driver comprising:
- a pushing element configured to cooperate with the plunger of the mini-syringe,
- a control unit configured to operate and control the pushing element in order to homogeneously empty the loaded mini-syringe according to pre-determined parameters decided by a user,
   o an activable securing and detection system presenting: a securing system comprising a restraining surface, the securing system being configured to receive and secure the mini-syringe along the injection axis and a perpendicular depth axis,
   o a detection system configured to detect the presence and the correct positioning of the mini-syringe inside the securing system, the detection system being configured to emit positive feedback when the mini-syringe is correctly secured,
**wherein** the control unit is configured to wait for positive feedback from the detection system for launching the operation of the pushing element.

This way, the solution enables to reach the here-above mentioned objective. Especially, it enables to safely and precisely inject, in a controlled way, very small fluid quantities (below 100 µL) repeatedly over time (from minutes to hours), which cannot be injected solely by a human user due to syringe volume errors and manual variations. The injection precision is thus increased by 5 to 20 times while being achieved in and in a repeatable way (need of precision around +/-3µL).

The system according to the invention may comprises one or several of the following features, taken separately from each other or combined with each other:
- the securing system may further comprise a locking element displaceable between:
   o an activated position in which a securing surface of the locking element cooperates with the external wall of the mini-syringe in order to maintain said mini-syringe on the restraining surface,
   o a resting position in which the securing surface is disengaged,
- the restraining surface may comprise a syringe positioning element configured to precisely position the mini-syringe along the injection axis and the depth axis,
- the syringe positioning element may be configured to position any mini-syringe at the same height along the depth axis, regardless of its width,
- the elongated syringe positioning element may be an elongated groove extending along the injection axis and presenting a pre-defined depth extending along the depth axis, thus enabling to precisely know the position of the received mini-syringe,
- the elongated groove may present staged walls in order to adapt to different mini-syringes presenting different widths, the position of the mini-syringe inside the elongated groove depending on the width of said mini-syringe and thus varying on the depth axis depending on the mini-syringe width,
- the elongated groove may present a V shape in order to adapt to different mini-syringes presenting different widths, the position of the mini-syringe inside the elongated groove depending on the width of said mini-syringe and thus varying on the depth axis depending on the mini-syringe width,
- the restraining surface may be removable from a body of the activable securing and detection system,
- the body of the activable securing and detection system may comprise a first recognition tag and the restraining surface the comprise a second recognition tag, the first and second recognition tags being configured to cooperate together thus enabling the restraining surface to be recognized by the securing and detection system,
- the detection system may be an optical sensor system configured to detect the height, along the depth axis, of a upper part of the external wall of the mini-syringe secured inside the securing member,
- the securing system and the detection system may both be activated by the same physical element,
- the securing system and the detection system may both be activated by the locking element,
- the detection system may further be configured to detect if the received mini-syringe is loaded or not,
- the mini-syringe may present a volume ranging from 0.5 to 5mL.

A further object of the present invention is a method for automatically emptying a loaded mini-syringe by means of a syringe driver according to any one of the technical features described here-above, the mini-syringe extending along an injection axis, the mini-syringe further comprising a plunger configured to translate inside the external wall and also extending along said injection axis, the method comprising following steps:
- positioning of the mini-syringe on the restraining surface along the injection axis,
- activation of the securing and detection system,
- detection of the positioned mini-syringe,
- emission of feedback,
- feedback analysis,
- in case of positive feedback, activation of the pushing element,
- axial cooperation, along the injection axis, with the plunger of the mini-syringe to empty said mini-syringe.

### BRIEF DESCRIPTION OF THE DRAWINGS

The invention will be better understood, and other aims, details, characteristics and advantages thereof will emerge more clearly on reading the detailed explanatory description which follows, of embodiments of the invention given by way of illustration, purely illustrative and non-limiting examples, with reference to the accompanying drawings:
- **figure 1** is schematic side view of a driver according to a first embodiment of the present invention,
- **figure 2** is schematic perspective view of the embodiment of figure 1,
- **figure 3** is a perspective view of a part of a driver according to a second embodiment,
- **figure 4a** is a schematic view of a syringe positioning element 20 according to a first embodiment,
- **figure 4b** is a schematic view of a syringe positioning element 20 according to a second embodiment,
- **figure 5** is a diagram illustration of a method implemented by means of a driver according to the present invention.

### DETAILED DESCRIPTION

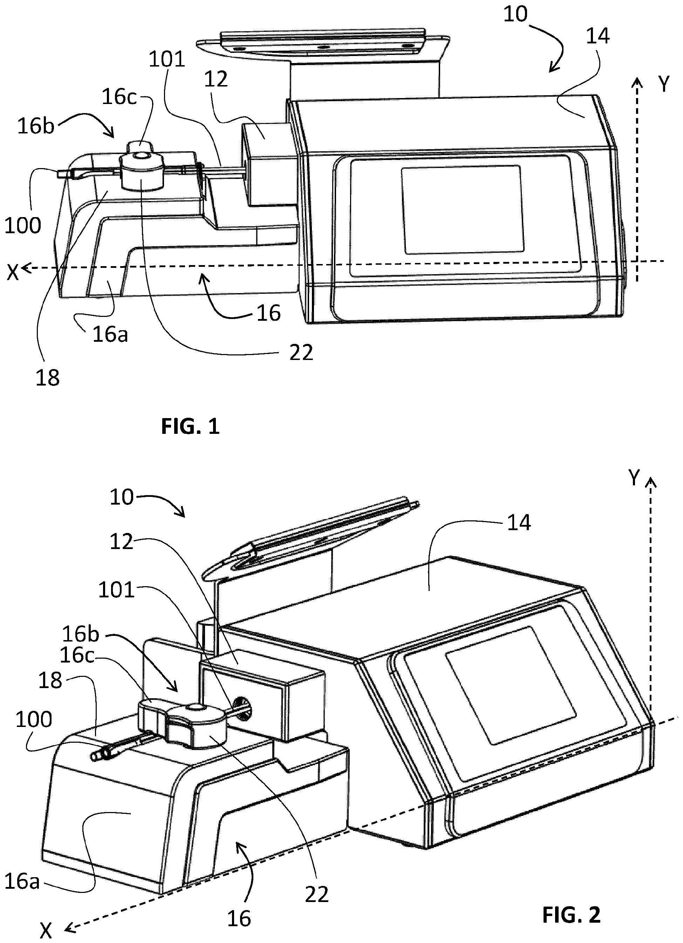

As can be seen on figures 1 and 2, this invention relates to a syringe driver 10 configured to empty, in an automatic way, a loaded mini-syringe 100.

The mini-syringe 100 is a standard mini-syringe extending along an injection axis X and presenting a body with an external wall with a diameter d ranging between 5mm and 15mm, more precisely ranging from 6 to 7mm for 1mL syringes. The mini-syringe 100 further classically comprises a plunger 101 configured to translate inside the external wall and also extending along said injection axis X. According to the definition of the present application the mini-syringe 100 presents a volume ranging below 5mL, preferably 1mL. Syringes are usually made of glass, or COC polymer or plastic. They are preferably single use.

As can be seen on figures 1 and 2, the driver 10 according to the present invention comprises:
- a pushing element 12 configured to cooperate with the plunger 101 of the mini-syringe 100,
- a control unit 14 configured to operate and control the pushing element 12,
- an activable securing and detection system 16.

The pushing element 12 presents a shape adapted to push the plunger 101 of the mini-syringe 100. As can be seen on figures 1 and 2, the pushing element 12 and plunger 101 of the syringe 100 are preferably colinear in order to improve devices precision.

In some embodiments, the pushing element 12 comprises a plunger 101 detection system with a first sensor which enables an automatic adjustment of the pushing element 12 to the position of the plunger 101without the need for manual securing, by a user, of the mini-syringe 100 to the driver 10. The first sensor can be a pressure sensor positioned inside the pushing element 12. The pushing element 12 may further comprise a second sensor which is able to detect if the minimal required engine stroke to enable both priming, or injection, or priming and injection. The second sensor can for example be a position switch, a potentiometer or a motor encoder.

The pushing element 12 may also comprises an anti-free flow system to lock the plunger 101 of the mini-syringe 100 and avoid free flow. Free flow designates the subtle back and forth movements of the plunger 101which can occur while pushing the plunger 101 forward or when the forward motion is interrupted for an injection break before the injection continues. In one embodiment, the anti-free flow system is made of three positions:
- one stable open position,
- one unstable intermediate position, and
- one closed stable position.
The unstable intermediate position enables the switch from the stable open to the stable closed position by means of a spring. The anti-free flow system has a detection sensor to inform the driver 10 if the securing and detection system 16 is activated. The detection sensor can be a micro-switch, or an optical system. The anti-free flow system can for example comprise a sliding door perpendicular to the injection axis X presenting a half circle to encircle the plunger 101 of the mini-syringe 100. The sliding door can be activated by a user thanks to a handle or can be automatically closed in response to a plunger 101 detection. Alternatively, the anti-free flow system is made of two rotating rod that encircle the plunger 101.

The activable securing and detection system 16 presents a body 16a with:
a. a securing system 16b comprising a restraining surface 18,
b. a detection system 16c.

The securing system 16b functions in a spatial referential comprising two perpendicular axes: the injection axis X and a depth axis Y. The origin of said referential is set and anchored to the body 16a of the securing system. The securing system 16b is thus configured to receive and tightly secure any mini-syringe 100 along the injection axis X and the perpendicular depth axis Y. It is preferably the body of the mini-syringe 100 which is secured by the securing system in order to reduce the risk of damaging the mini-syringe 100.

More particularly, it is the restraining surface 18 of the securing system 16b which is configured to receive the mini-syringe 100. Therefore, the restraining surface 18 comprises a syringe positioning element 20 configured to precisely position any mini-syringe 100 along the injection axis X and the depth axis Y. When recognizing the mini-syringe 100, it is important to differentiate the size of the mini-syringe 100 with high accuracy in order to accurately differentiate it from the other mini-syringes 100.

Preferably, the restraining surface 18, by means of the syringe positioning element 20, also immobilizes any positioned mini-syringe 100 along the axis X and preferably along the axis X and Y. As the precision of the mini-syringes 100 is highly sensitive to placement variation, for example induced by undesired movements of the driver 10, it is important to hold and secure all the elements of the mini-syringe 100 to guaranty great precision less than 5% dose variation.

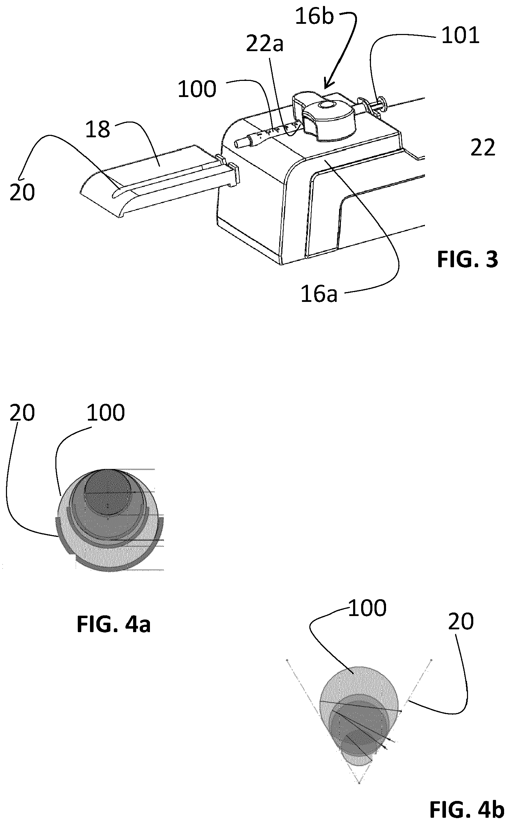

In order to enable this immobilization and according to the embodiment of figure 3, the syringe positioning element 20 can for example be an elongated groove extending along the injection axis X and presenting a pre-defined depth D extending along the depth axis Y. This elongated groove presents dimensions adapted to each of the different mini-syringe 100 to be received and secured by the driver 10. This tight adjustment of the mini-syringe 100, enables tolerance improvement and the avoidance of undesired parasitic movement which could affect the injection precision. This precise dimensioning of the elongated groove also enables to precisely know the position of the received mini-syringe 100. In some alternative embodiment (not shown), the syringe positioning element 20 might be a clamping system or a reversibly sticking system.

One of the interesting technical possibilities of the driver 10 according to the present invention, is its capacity to be adapt to a series of different mini-syringes 100 without affecting the precision of the automatic emptying of each of these loaded mini-syringes 100. In this regard, the driver 10 according to the present invention can present, among others, three possible technical solutions:
- in a first embodiment, the restraining surface 18 is removable from the body 16a of the activable securing and detection system 16 (see figure 3),
- in a second embodiment (see figures 4a and 4b), the restraining surface 18, and more particularly the syringe positioning element 20, presents an evolutive shape which enables each possible mini-syringe 100 to fit inside the driver 10,
- in a third embodiment (not shown), the restraining surface 18, and more particularly the syringe positioning element 20, presents an adaptable shape to each mini-syringe 100 to be fitted inside the driver 10,

Regarding the first embodiment (see figure 3), the restraining 18 surface is a separate technical element from the body 16a of the activable securing and detection system 16. To each possible mini-syringe 100 thus corresponds a different restraining surface 18. For example, each restraining surface 18 presents syringe positioning element 20, for example an elongated groove, specifically dimensioned to fit one specific mini-syringe 100. Each of these surfaces can be secured to the body 16a of the activable securing and detection system 16 in order to enable the securing of the corresponding mini-syringe 100 inside the driver 10. In this case, the restraining surface is preferably part of an easy-to-handle technical element, for example a square shaped element. The body 16a of the activable securing and detection system 16 and the restraining surface 18 each present corresponding connecting and securing means configured to safely and reversibly connect and secure the restraining surface 18 to the body 16a of the activable securing and detection system 16. The position of the mini-syringe 100 on the restraining surface 18 is thus specific of the cooperation of the mini-syringe 100 with its corresponding matching restraining surface 18.

In order to improve security, the body 16a of the activable securing and detection system 16 comprises a first recognition tag and the restraining surface 18 comprises a second recognition tag. Those first and second recognition tags can for example be a QR code and a QR code detector or a physical embeddable part with a specific design. Those first and second recognition tags are configured to cooperate together in order to enable the restraining surface 18 to be recognized by the securing and detection system 16. The securing and detection system 16 can thus adapt to each specific restraining surface 18. Those recognition tags further enable the automatic calibration of the control unit 14 and the determination of key parameters such as recognition of a plunger stroke and syringe diameter. An additional tag can include treatment references in order to preselect administration parameters, thus avoiding any possible misuse of the system.

Considering the second embodiment, the syringe positioning element 20, for example an elongated groove, can present staged walls in order to adapt to different mini-syringes 100 presenting different widths. In some alternative embodiment, as can be seen on figure 4b, the syringe positioning element 20, for example an elongated groove, presents a V shape in order to adapt to different mini-syringes 100 presenting different widths. The position of each different mini-syringe 100 inside the elongated groove thus depends on the width of said each mini-syringe 100 and thus varies on the depth Y axis depending on the mini-syringe width. Depth shall at least be equal to 30% of the outer diameter of the mini-syringe 100, preferably 50%, and more preferably 75%. The outer diameter of the mini-syringe 100 is below 8mm, depending on the syringe brand with a two-digit precision.

In some other embodiments (not shown), the syringe positioning element 20, for example an elongated groove, is configured to position any mini-syringe at the same height along the depth axis Y, regardless of its width.

Regardless of the embodiment, the precise position of each mini-syringe 100 according to the injection axis X and the depth axis Y are well known.

In order to stabilize the mini-syringe 100 and to further improve its immobilization for an improved security in the use of the driver 10 according to the present invention, the securing system 16b further comprises a locking element 22 displaceable between:
- an activated position in which a securing surface 22a of the locking element 22 cooperates with the external wall of the mini-syringe 100, preferably the body of the mini-syringe 100, in order to maintain it on the restraining surface 18,
- a resting position in which the securing surface 18 is disengaged.

The resting position thus permits to insert or remove the mini-syringe from the restraining surface 18 and the syringe positioning element 20.

The locking element 22 can for example be a pull and rotate system, a clamping system such as a bracket. The locking element is preferably easy to activate in order to facilitate the driver handling and limit the human manipulation prior to injection.

In order to enable a safe automated emptying of the loaded mini-syringe 100 secured to the securing system 16b of the driver 10, the securing system 16b cooperates with the detection system 16c for each inserted mini-syringe 100.

More exactly, the detection system 16c is configured to detect the presence and the correct positioning of each mini-syringe 100 inside the securing system 16b. The detection system 16c enables the driver 10 according to the present invention to recognize a specific mini-syringe reference charactrised by its outershape and its outer diameter.

The detection system 16c can for example be an optical sensor system configured to detect the height, along the depth axis Y, of an upper part of the external wall of the mini-syringe 100 secured on the restraining surface 18.

This optical sensor system can for example be an optic fork sensor. An optical fork sensor is known, in the state of the art, to detect objects passing between two arms, one being the transmitter and the other the receiver.

In some embodiments, the detection system 16c is at least partially comprised within the syringe positioning element 20. In those embodiments, the syringe positioning element 20 comprises syringe detection means. Those means can comprise a sensor integrated within the walls of the positioning element 20. When the locking element 22 is put in its activated position, the locking element 22 presses on the syringe and thus indirectly applies pressure on the sensors in the positioning element 20 which enables the detection of the inserted mini-syringe 100.

If the secured mini-syringe 100 is not correctly immobilized on the restraining surface 18 or if the detected height does not match with a pre-recorded value, or if the detected height does not align with the expected height in case all the mini-syringes 100 are to be positioned at the same height along the depth axis Y, or if a removable restraining surface 18 is not correctly secured to the body 16a of the securing and activation system 16, the injection cannot start and the pushing element 12 remains immobile.

In some alternative embodiment, the detection system 16c is further configured to detect if the received mini-syringe 100 is loaded or not. This can for example be achieved by a weighing system comprised within the detection system 16c or, or by a pressure measuring system, or by another optical detection system, for example photometry measures. If the mini-syringe is not detected to be correctly leaded, the injection cannot start and the pushing element 12 remains immobile.

The detection system 16c is configured to emit positive feedback when the mini-syringe 100 is correctly secured to the restraining surface 18. In some embodiments, the detection system 16c is configured to emit positive feedback when the mini-syringe 100 is correctly secured to the restraining surface and correctly loaded. Without this positive feedback, nothing happens. The control unit 14 is configured to wait for positive feedback from the detection system 16c for launching the operating of the pushing element 12. The operating of the pushing element 12 comprises two phases: an engagement phase and an emptying phase. During the engagement phase, the pushing element 12 is put in forward motion and moves on its own till it reaches the plunger 101 of the mini-syringe 100. Contact between the pushing element 12 and the plunger 101 is thus established after the engagement phase. This contact enables the activation of the hare-above mentioned anti-free flow system to lock the plunger 101 or of the different here-above mentioned sensors present in the pushing element 12. During the emptying phase, the pushing element 12 is put in forward motion and moves along with the plunger 101 in order to empty the mini-syringe 100. More precisely, the pushing element 12 puts the plunger 101 in forward motion. The emptying phase can comprise a priming phase and an actual injection phase.

In order to be sure that the mini-syringe has been correctly secured to the restraining surface 18 of the securing system 16b by the user before the detection system 16c starts its measures, in some embodiments, the securing system 16b and the detection system 16c are both activated by the same physical element. This way, a user can calmy insert the mini-syringe 100 and once finished, activate both the securing system 16b (in case the syringe positioning element 20 comprises a clamping system, for example) and the detection system 16c by activating/moving/pushing one single physical element. In case the driver 10 comprises a locking element 22, the securing system 16b and the detection system 16c are both activated by the locking element 22. This means that when the user displaces the locking element 22 from its resting position to its activated position, the detection system 16c starts measuring the position (and, if relevant and possible, the load) of the inserted mini-syringe 100.

The communication between all the elements of the driver 10 according to the present invention is ensured by the control unit 14. More specifically, the control unit 14 presents a casing and comprises a computing device.

The control unit 14 further comprises an interaction interface (for example a screen and/or a keyboard) which enables a user to enter and/or pre-record parameter which determine the injection. The control unit 14 thus enables the driver 10 according to the present invention, to homogeneously empty any correctly inserted loaded mini-syringe 100 according to pre-determined parameters decided by a user.

The control unit 14 is configured to enable the control and administration of a constant flowrate with no pulsatile effect due to motorization. The flow rate range can be fixed (pre-programed) of adjustable from 1µL.min to 4000 µL.min.

In some embodiments, the control unit 14 may also detect, through the pushing element 12, an occlusion happening inside the mini-syringe 100 or a catheter connected to the mini-syringe 100. In case such an occlusion is present, the pushing element 12 may have to provide an augmented injection pressure which can be interpreted, by the control unit 14, as the present of an occlusion and could start an emergency program. As a preventive response to pressure increase, an automatic backward movement can be triggered to release said pressure.

The user might then be able to follow the injection process by means of a screen.

The driver 10 according to the present application enables the implementation of a method for automatically emptying a loaded mini-syringe 100.

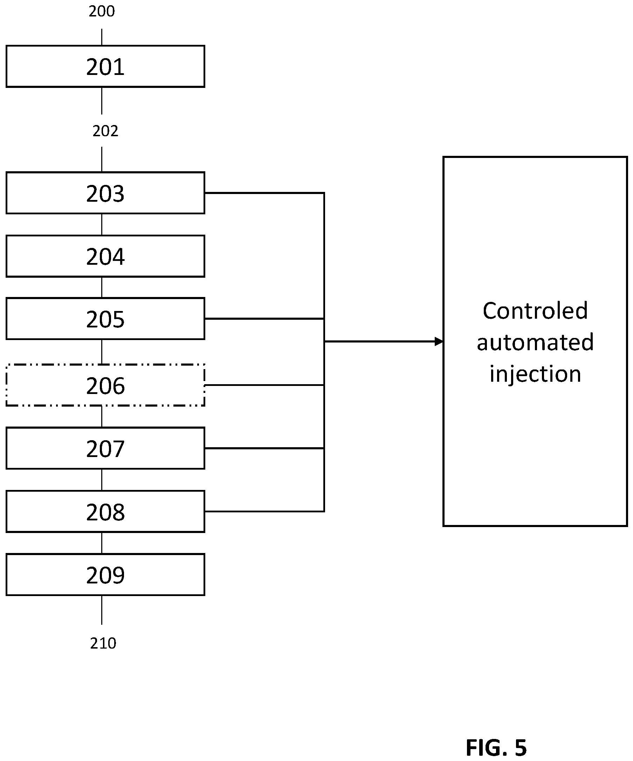

On the diagram of figure 5, the automated steps are represented in a square and the few steps operated by the user are represented outside a square. The optional steps are represented in a dotted square.

The method comprises following steps:
- positioning 202 of the mini-syringe 100 on the restraining surface 18 along the injection axis X,
- if necessary, securing the restraining surface 18 to the body 16a of the activable securing and detection system 16,
- activation 203 of the securing and detection system 16, if possible, by displacing the locking element 22
- detection 204 and, depending of the embodiment, recognition of the positioned mini-syringe 100,
- if possible, detection of load of the mini-syringe 100,
- emission of feedback from the detection system 16c to the control unit 14,
- feedback analysis by the control unit 14,
- in case of positive feedback, activation, by the control unit 14, of the pushing element 12, in order to start the engagement and emptying phases,
- axial cooperation 208, along the injection axis X, with the plunger 101 of the mini-syringe 100 to empty said mini-syringe 100.

Prior to the positioning 202 of the mini-syringe 100, the user switches the driver on 200 (by pushing a start button, for example) followed by an initialization 201 of an internal informatic system of the control unit 14.

Depending on the embodiment, before the activation 204, by the control unit 14, of the pushing element 12, the driver 10 may detect 205, after the engagement phase, the plunger 101 and activate 206 the anti-free flow system. This enables an automatic connection between the pushing element 12 and the plunger 101 avec avoids any manual human intervention.

After the injection has been completed, the securing system 16b unlocks 209 the mini-syringe 100 and the pushing element 12 unlocks the plunger 101 and the mini-syringe 100 has to be removed 210 by the user and the driver 10 has to be switched off.

The motion of the pushing element 12 might lead to a priming step 207 before the actual injection step 208. This might be particularly relevant in case the mini-syringe 100 is connected to a long catheter before reaching the patient in order to avoid the injection of air bubbles in the injection sites.

The present invention enables to provide a mini-syringe driver 10 capable of avoiding injections based on erroneous syringe determination, while maximally reducing human interactions by guarantying positioning and recognition in a single action. Human handling is a possible source of errors and imprecisions, and further an possible source of microbic contamination or drug leakage. It is therefore a significant increase in safety and precision to reduce as much as possible the human interaction with the mini-syringe 100 during all the injection process. The present invention thus successfully enables reducing handling of the mini-syringe thanks to an automated sequence of action launched after the syringe recognition and securing.

## Claims

1. Syringe driver (10) configured to empty, in an automatic way, a loaded mini-syringe (100) extending along an injection axis (X) and presenting an external wall with a diameter d, the mini-syringe (100) further comprising a plunger (101) configured to translate inside the external wall and also extending along said injection axis (X), said driver (10) comprising:
- a pushing element (12) configured to cooperate with the plunger (101) of the mini-syringe (100),
- a control unit (14) configured to operate and control the pushing element (12) in order to homogeneously empty the loaded mini-syringe (100) according to pre-determined parameters decided by a user,
o an activable securing and detection system (16) presenting: a securing system (16b) comprising a restraining surface (18), the securing system (16b) being configured to receive and secure the mini-syringe (100) along the injection axis (X) and a perpendicular depth axis (Y),
o a detection system (16c) configured to detect the presence and the correct positioning of the mini-syringe (100) inside the securing system (16b), the detection system (16c) being configured to emit positive feedback when the mini-syringe (100) is correctly secured,
**wherein** the control unit (14) is configured to wait for positive feedback from the detection system (16b) for launching the operation of the pushing element (12).

2. Syringe driver (10) according to the preceding claim, **wherein** the securing system (16b) further comprises a locking element (22) displaceable between:
- an activated position in which a securing surface (22a) of the locking element cooperates with the external wall of the mini-syringe (100) in order to maintain said mini-syringe (100) on the restraining surface (18),
- a resting position in which the securing surface (22a) is disengaged.

3. Syringe driver (10) according to any one of the preceding claims, **wherein** the restraining surface (18) comprises a syringe positioning element (20) configured to precisely position the mini-syringe (100) along the injection axis (X) and the depth axis (Y).

4. Syringe driver (10) according to the preceding claim, **wherein** the syringe positioning element (20) is configured to position any mini-syringe (100) at the same height along the depth axis (Y), regardless of its width.

5. Syringe driver (10) according to any one of the preceding claims, **wherein** the elongated syringe positioning element (20) is an elongated groove extending along the injection axis (X) and presenting a pre-defined depth (D) extending along the depth axis (Y), thus enabling to precisely know the position of the received mini-syringe (100).

6. Syringe driver (10) according to the preceding claim, **wherein** the elongated groove presents staged walls in order to adapt to different mini-syringes (100) presenting different widths, the position of the mini-syringe (100) inside the elongated groove depending on the width of said mini-syringe (100) and thus varying on the depth axis (Y) depending on the mini-syringe width.

7. Syringe driver (10) according to claim **5, wherein** the elongated groove presents a V shape in order to adapt to different mini-syringes (100) presenting different widths, the position of the mini-syringe (100) inside the elongated groove depending on the width of said mini-syringe (100) and thus varying on the depth axis (Y) depending on the mini-syringe width.

8. Syringe driver (10) according to any one of the preceding claims, **wherein** the restraining surface (18) is removable from a body (16a) of the activable securing and detection system (16).

9. Syringe driver (10) according to the preceding claim, **wherein** the body (16a) of the activable securing and detection system (16) comprises a first recognition tag and the restraining surface the comprise a second recognition tag, the first and second recognition tags being configured to cooperate together thus enabling the restraining surface (18) to be recognized by the securing and detection system (16).

10. Syringe driver according to any one of the preceding claims, **wherein** the detection system is an optical sensor system configured to detect the height, along the depth axis (Y), of a upper part of the external wall of the mini-syringe secured inside the securing member.

11. Syringe driver (10) according to any one of the preceding claims, **wherein** the securing system (16b) and the detection system (16c) are both activated by the same physical element.

12. Syringe driver (10) according to claim **2, wherein** the securing system (16b) and the detection system (16c) are both activated by the locking element (22).

13. Syringe driver (10) according to any one of the preceding claims, **wherein** the detection system (16b) is further configured to detect if the received mini-syringe (100) is loaded or not.

14. Syringe driver (10) according to any one of the preceding claims, **wherein** the mini-syringe (100) presents a volume ranging from 0.5 to 5mL.

15. Method for automatically emptying a loaded mini-syringe (100) by means of a syringe driver (10) according to any one of the preceding claims, the mini-syringe (100) extending along an injection axis (X), the mini-syringe (100) further comprising a plunger (101) configured to translate inside the external wall and also extending along said injection axis (X), the method comprising following steps:
- positioning of the mini-syringe (100) on the restraining surface (18) along the injection axis (X),
- activation of the securing and detection system (16),
- detection of the positioned mini-syringe (100),
- emission of feedback,
- feedback analysis,
- in case of positive feedback, activation of the pushing element (12),
- axial cooperation, along the injection axis (X), with the plunger (101) of the mini-syringe (100) to empty said mini-syringe (100).
